# EUROPÄISCHE PATENTANMELDUNG

(11) **EP 3 751 523 A1**
(43) Veröffentlichungstag der Anmeldung: **16.12.2020**
(21) Anmeldenummer: 20179924.4
(22) Anmeldetag: 15.06.2020
(51) Int. Cl.: G06T 19/00, A61B 1/05, H04N 21/643, H04N 19/00

(54) **VIDEOBILD-ÜBERTRAGUNGSEINRICHTUNG, VERFAHREN ZUR LIVE-ÜBERTRAGUNG UND COMPUTERPROGRAMM**

(30) Priorität: 14.06.2019 DE 102019116269
(71) Anmelder: apoQlar GmbH, 20099 Hamburg (DE)
(72) Erfinder: Sirko Pelzl, 20146 Hamburg (DE); Pawel Skiba, 61-643 Poznan (PL)
(74) Vertreter: Gramm, Lins & Partner Patent- und Rechtsanwälte PartGmbB

(57) **Zusammenfassung**

Die Erfindung betrifft eine Videobild-Übertragungseinrichtung (2), ein zugehöriges Verfahren und ein zugehöriges Computerprogramm zur Live-Übertragung von Videobildsequenzen mittels Streaming von einer Videobildquelle auf eine Augmented Reality-, Virtual Reality- oder Mixed Reality-Anzeigeeinrichtung (9), wobei die Videobild-Übertragungseinrichtung (2)
a) einen Eingang (3) aufweist, der zur Zuführung zu übertragender Videobildsequenzen von einer Videobildquelle (1) zu einer Verarbeitungseinrichtung (4) der Videobild-Übertragungseinrichtung eingerichtet ist,
b) die Verarbeitungseinrichtung (4) aufweist, die zur Live-Umwandlung der über den Eingang empfangenen Videobildsequenzen in auszugebende Videobildsequenzen eingerichtet ist,
c) einen Ausgang (5) aufweist, der zur Ausgabe der auszugebenden Videobildsequenzen an die Augmented Reality-, Virtual Reality- oder Mixed Reality-Anzeigeeinrichtung (9) eingerichtet ist,
wobei der Eingang (3) dazu eingerichtet ist, die zu übertragenden Videobildsequenzen von einem bildgebenden medizinischen Untersuchungsgerät als Videobildquelle (1) zu empfangen

## Beschreibung

Die Erfindung betrifft eine Videobild-Übertragungseinrichtung zur Live-Übertragung von Videobildsequenzen mittels Streaming (mit geringer Latenz). Die Erfindung betrifft außerdem ein Verfahren zur Live-Übertragung von Videobildsequenzen mittels Streaming sowie ein Computerprogramm zur Durchführung eines derartigen Verfahrens.

Die Live-Übertragung von Videobildsequenzen mittels Streaming ist im Multimediabereich weit verbreitet, z.B. für die Übertragung von Fernsehprogrammen über das Internet oder für die Bildübertragung bei Videokonferenzen. Die Übertragung der Videobildsequenzen erfolgt dabei praktisch in Echtzeit, wobei in der Praxis aufgrund von Datenübertragungszeiten und Rechenzeiten zwischengeschalteter Computereinrichtungen eine geringfügige zeitliche Divergenz auftreten kann, die aber für die praktische Nutzung unbedeutend ist. Beim Streaming wird eine fortlaufende Videobildsequenz permanent übertragen, ohne dass zunächst auf den Abschluss der Übertragung der gesamten Videobildsequenz gewartet wird und diese erst dann vollständig aus einem Speicher abgespielt wird.

Aus der EP 3 167 616 B1 ist ein Verfahren zum Kodieren von Videodaten für eine drahtlose Übertragung bekannt, bei dem mittels eines Video-Codecs ein Segment eines Quellvideos basierend auf einer Kodierungsbitrate kodiert wird. Als Kodierungsbitrate wird dabei das Minimum aus drei verschiedenen in Betracht kommenden Bitraten ausgewählt.

Der Erfindung liegt die Aufgabe zugrunde, eine für den Anwender einfach zu handhabende Lösung für die Live-Übertragung von Videobildsequenzen mittels Streaming anzugeben.

Diese Aufgabe wird gelöst durch eine Videobild-Übertragungseinrichtung zur Live-Übertragung von Videobildsequenzen mittels Streaming mit den Merkmalen des Anspruchs 1.

Erfindungsgemäß ist vorgesehen, dass die Videobild-Übertragungseinrichtung
a) einen Eingang aufweist, der zur Zuführung zu übertragender Videobildsequenzen von einer Videobildquelle zu einer Verarbeitungseinrichtung der Videobild-Übertragungseinrichtung eingerichtet ist,
b) die Verarbeitungseinrichtung aufweist, die zur Live-Umwandlung der über den Eingang empfangenen Videobildsequenzen in auszugebende Videobildsequenzen eingerichtet ist, und
c) einen Ausgang aufweist, der zur Ausgabe der auszugebenden Videobildsequenzen an die Augmented Reality-, Virtual Reality- oder Mixed Reality-Anzeigeeinrichtung Videobildsequenz-Anzeigegerät eingerichtet ist.
wobei der Ausgang für die Ausgabe der auszugebenden Videobildsequenzen auf einer Augmented Reality-, Virtual Reality- oder Mixed Reality-Anzeigeeinrichtung eingerichtet ist und der Eingang dazu eingerichtet ist, die zu übertragenden Videobildsequenzen von einem bildgebenden medizinischen Untersuchungsgerät als Videobildquelle zu empfangen.

In ähnlicher Weise kann vorgesehen sein, dass die Videobild-Übertragungseinrichtung
a) einen Eingang aufweist, der zur Zuführung zu übertragender Videobildsequenzen von einer Videobildquelle zu einer Verarbeitungseinrichtung der Videobild-Übertragungseinrichtung eingerichtet ist,
b) die Verarbeitungseinrichtung aufweist, die zur Live-Umwandlung der über den Eingang empfangenen Videobildsequenzen in auszugebende Videobildsequenzen eingerichtet ist, und
c) einen Ausgang aufweist, der zur Ausgabe der auszugebenden Videobildsequenzen an ein Videobildsequenz-Anzeigegerät eingerichtet ist,
wobei der Ausgang für die Ausgabe der auszugebenden Videobildsequenzen auf einer Augmented Reality-, Virtual Reality- oder Mixed Reality-Anzeigeeinrichtung eingerichtet ist.

Vorteilhaft wird hiermit eine Plug-and-Play-Lösung für die Live-Übertragung von Videobildsequenzen von einer praktisch beliebigen Videobildquelle auf eine Augmented Reality-, Virtual Reality- oder Mixed Reality-Anzeigeeinrichtung (nachfolgend kurz AR-Anzeigeeinrichtung genannt) angegeben. Damit betrifft die Erfindung das Gebiet der Visualisierung virtueller Realität in Kombination mit der realen Umgebung. Dieses Gebiet wird auch als Augmented Reality-, Virtual Reality- oder Mixed Reality-Darstellung bezeichnet. Die verschiedenen Begriffe werden zum Teil für gleiche Arten der Darstellung genutzt. Es zeichnet sich ab, dass der Begriff der Mixed Reality zunehmend für Anwendungen verwendet wird, bei denen im Unterschied zu Augmented Reality eine Interaktion des Benutzers mit den virtuell dargestellten Objekten möglich ist, z.B. indem durch manuelle Bewegungen des Benutzers eine Änderung der Darstellung erfolgen kann. In beiden Fällen, d.h. bei Augmented Reality und Mixed Reality, wird realen Objekten auf einem Display einer Anzeigeeinrichtung eine virtuelle Information überlagert.

Die AR-Anzeigeeinrichtung kann z.B. eine Videobrille sein, beispielsweise die HoloLens von Microsoft. Bisher gibt es hierzu noch keine für den Anwender einfach zu handhabende Lösung, um Videobildsequenzen von einer beliebigen Videobildquelle, z.B. von einem bildgebenden medizinischen Untersuchungsgerät, auf eine solche AR-Anzeigeeinrichtung zu übertragen. Die erfindungsgemäße Videobild-Übertragungseinrichtung kann dabei hinsichtlich ihres Hardware-Aufbaus und ihrer Dimensionen einfach und kompakt gehalten werden, z.B. als handliche kleine Box, die vom Anwender leicht transportiert werden kann und ohne besondere Vorkenntnisse dann für die Live-Übertragung der Videobildsequenzen eingesetzt werden kann.

Die Videobild-Übertragungseinrichtung kann ein Gehäuse aufweisen. In das Gehäuse können die Komponenten der Videobild-Übertragungseinrichtung, insbesondere der Eingang, die Verarbeitungseinrichtung und der Ausgang, integriert sein.

Der Eingang der Videobild-Übertragungseinrichtung kann dazu eingerichtet sein, mit der Videobildquelle, insbesondere mit dem bildgebenden medizinischen Untersuchungsgerät, gekoppelt zu werden. Der Eingang der Videobild-Übertragungseinrichtung kann insbesondere dazu eingerichtet sein, über eine lösbare Kabelverbindung mit der Videobildquelle, insbesondere mit dem bildgebenden medizinischen Untersuchungsgerät, gekoppelt und/oder verbunden zu werden. Alternativ oder ergänzend hierzu kann der Eingang der Videobild-Übertragungseinrichtung auch dazu eingerichtet sein, über eine drahtlose Verbindung, z. B. über eine Funkverbindung, mit der Videobildquelle, insbesondere mit dem bildgebenden medizinischen Untersuchungsgerät, gekoppelt und/oder verbunden zu werden. Der Eingang kann in diesem Fall als drahtlose Schnittstelle, insbesondere als Funkschnittstelle, ausgebildet sein und/oder eine solche drahtlose Schnittstelle, insbesondere eine Funkschnittstelle, aufweisen. Die Funkschnittstelle kann z. B. als WLAN-Schnittstelle ausgebildet sein.

Die Videobildquelle, insbesondere das bildgebende medizinische Untersuchungsgerät, kann dabei von der Videobildübertragungseinrichtung separat sein. Die Videobildquelle, insbesondere das bildgebende medizinische Untersuchungsgerät, kann dabei insbesondere außerhalb des Gehäuses der Videobild-Übertragungseinrichtung angeordnet sein.

Der Ausgang der Videobild-Übertragungseinrichtung kann dazu eingerichtet sein, mit der AR-Anzeigeeinrichtung gekoppelt zu werden. Der Ausgang der Videobild-Übertragungseinrichtung kann insbesondere dazu eingerichtet sein, über eine lösbare Kabelverbindung mit der AR-Anzeigeeinrichtung gekoppelt und/oder verbunden zu werden. Alternativ oder ergänzend hierzu kann der Ausgang der Videobild-Übertragungseinrichtung auch dazu eingerichtet sein, über eine drahtlose Verbindung, z. B. über eine Funkverbindung, mit der AR-Anzeigeeinrichtung gekoppelt und/oder verbunden zu werden. Der Ausgang kann in diesem Fall als drahtlose Schnittstelle, insbesondere als Funkschnittstelle, ausgebildet sein und/oder eine solche drahtlose Schnittstelle, insbesondere eine Funkschnittstelle, aufweisen. Die Funkschnittstelle kann z. B. als WLAN-Schnittstelle ausgebildet sein.

Die AR-Anzeigeeinrichtung kann dabei von der Videobildübertragungseinrichtung separat sein. Die AR-Anzeigeeinrichtung kann dabei insbesondere außerhalb des Gehäuses der Videobild-Übertragungseinrichtung angeordnet sein.

Der Eingang der Videobild-Übertragungseinrichtung kann ein Eingang für die drahtgebundene Übertragung von Videobildsequenzen sein, beispielsweise ein HDMI-Anschluss. Durch entsprechende Hardware-Gestaltung des Eingangs und entsprechender Teile der Verarbeitungseinrichtung für die Verarbeitung der über den Eingang empfangenen Videobildsequenzen ist eine spezifische Adaption der Videobild-Übertragungseinrichtung an bestimmte Arten von Videobildquellen möglich, z.B. an Videobildquellen aus dem medizinischen Bereich, z.B. bildgebende medizinische Untersuchungsgeräte wie ein Endoskop, Ultraschallgerät, Röntgengerät oder ein Mikroskop.

Der Ausgang der Videobild-Übertragungseinrichtung kann ein Ausgang für die drahtgebundene oder die drahtlose Übertragung von Videobildsequenzen sein, beispielsweise ein HDMI-Anschluss oder eine WLAN-Schnittstelle.

Die Verarbeitungseinrichtung kann einen Rechner aufweisen, durch den die Live-Umwandlung der über den Eingang empfangenen Videobildsequenzen in auszugebende Videobildsequenzen ausgeführt wird. Der Rechner kann z.B. ein Computerprogramm ausführen, durch das diese Live-Umwandlung erfolgt. Durch alle diese Maßnahmen kann die Live-Übertragung mittels Streaming optimiert werden. Die Verarbeitungseinrichtung kann einen Decoder zur Decodierung der empfangenen Videobildsequenzen und einen Encoder zur Codierung der auszugebenden Videobildsequenzen aufweisen.

Gemäß einer vorteilhaften Ausgestaltung der Erfindung ist vorgesehen, dass der Ausgang als drahtlose Schnittstelle, insbesondere als WLAN-Schnittstelle, ausgebildet ist. Dies hat den Vorteil, dass ausgangsseitig keine Verdrahtung der Videobild-Übertragungseinrichtung erforderlich ist, wodurch die Einsatzmöglichkeiten der Videobild-Übertragungseinrichtung weiter verbessert werden. Dies hat zudem den Vorteil, dass auch die AR-Anzeigeeinrichtung, der die auszugebenden Videobildsequenzen zugeführt werden, nicht drahtgebunden mit der Videobild-Übertragungseinrichtung verbunden werden muss, sondern ebenfalls über die drahtlose Schnittstelle gekoppelt werden kann. Auf diese Weise wird die Bewegungsfreiheit des Nutzers der AR-Anzeigeeinrichtung nicht eingeschränkt. Insbesondere kann die Videobild-Übertragungseinrichtung als ein Netzwerkteilnehmer in einem drahtlosen Netzwerk, z.B. einem WLAN-Netzwerk, ausgebildet sein.

Gemäß einer vorteilhaften Ausgestaltung der Erfindung ist vorgesehen, dass die Verarbeitungseinrichtung wenigstens einen Codierungskonverter aufweist, der dazu eingerichtet ist, über den Eingang empfangene Videobildsequenzen einer ersten Codierung in auszugebende Videobildsequenzen einer zweiten Codierung, die sich von der ersten Codierung unterscheidet, zu konvertieren. Dies ermöglicht einen flexiblen Einsatz der Videobild-Übertragungseinrichtung mit jeweiligen unterschiedlichen Videobildquellen. Die Videobild-Übertragungseinrichtung kann auch mehrere Codierungskonverter aufweisen, um die Codierungen unterschiedlicher Arten von Videobildquellen in die zweite Codierung zu konvertieren.

Gemäß einer vorteilhaften Ausgestaltung der Erfindung ist vorgesehen, dass die Videobild-Übertragungseinrichtung wenigstens ein Display und eine Displayansteuerung aufweist, die dazu eingerichtet ist, auf dem Display Betriebsdaten der Videobild-Übertragungseinrichtung zu visualisieren. Dies hat den Vorteil, dass dem Anwender der Videobild-Übertragungseinrichtung auf einfache Weise der aktuelle Betriebs- und Funktionsstatus der Videobild-Übertragungseinrichtung visualisiert werden kann. Dies ist beispielsweise hilfreich, wenn zunächst die Drahtlos-Datenverbindung zu der AR-Anzeigeeinrichtung hergestellt werden soll. Beispielsweise können auf dem Display dann die Netzwerk-Adressen der in der Umgebung verfügbaren Geräte, die die drahtlose Datenverbindung unterstützen, angezeigt werden.

Gemäß einer vorteilhaften Ausgestaltung der Erfindung ist vorgesehen, dass die Displayansteuerung dazu eingerichtet ist, auf dem Display die eigene Netzwerkadresse der Videobild-Übertragungseinrichtung und/oder eine Netzwerk-Adresse der Augmented Reality-, Virtual Reality- oder Mixed Reality-Anzeigeeinrichtung, auf die auszugebenden Videobildsequenzen übertragen werden sollen, im Klartext und/oder in einer maschinenlesbaren Codierung zu visualisieren. Dabei kann dem Anwender zusätzlich auf dem Display angezeigt werden, um welche Art von Gerät es sich handelt, beispielsweise um die AR-Anzeigeeinrichtung. Auch dies vereinfacht die Herstellung der gewünschten Datenverbindung der Videobild-Übertragungseinrichtung mit der AR-Anzeigeeinrichtung für den Anwender. Wird die Netzwerk-Adresse in einer maschinenlesbaren Codierung visualisiert, ist es für den Anwender besonders einfach, die Datenverbindung zur AR-Anzeigeeinrichtung herzustellen, da mittels der AR-Anzeigeeinrichtung diese maschinenlesbare Codierung vom Display der Videobild-Übertragungseinrichtung gescannt und automatisch ausgewertet werden kann.

Erfindungsgemäß ist vorgesehen, dass der Eingang dazu eingerichtet ist, Videobildsequenzen von einem bildgebenden medizinischen Untersuchungsgerät zu empfangen. Das bildgebende medizinische Untersuchungsgerät kann insbesondere ein Endoskop oder ein Mikroskop sein. Dementsprechend ist die Videobild-Übertragungseinrichtung speziell dazu eingerichtet, mit bildgebenden medizinischen Untersuchungsgeräten zusammenzuwirken. Auf diese Weise kann die Nutzung von Augmented Reality-, Virtual Reality- oder Mixed-Reality-Anwendungen im medizinischen Bereich gefördert werden.

Die eingangs genannte Aufgabe wird außerdem gelöst durch ein Verfahren zur Live-Übertragung von Videobildsequenzen mittels Streaming von einer Videobildquelle auf eine Augmented Reality-, Virtual Reality- oder Mixed Reality-Anzeigeeinrichtung unter Verwendung einer Videobild-Übertragungseinrichtung der zuvor erläuterten Art. Auch hierdurch können die zuvor erläuterten Vorteile realisiert werden. Hierbei wird die Videobild-Übertragungseinrichtung mit ihrem Eingang mit der Videobildquelle gekoppelt, und mit ihrem Ausgang mit der AR-Anzeigeeinrichtung.

Gemäß einer vorteilhaften Ausgestaltung der Erfindung ist vorgesehen, dass die Übertragung der Videobildsequenzen von der Videobildquelle zum Eingang und/oder vom Ausgang zu der Augmented Reality-, Virtual Reality- oder Mixed Reality-Anzeigeeinrichtung im VSI-Format (VSI - Virtual Surgery Intelligence) erfolgt. Dies erlaubt eine besonders effiziente Übertragung der Videobildsequenzen im Bereich medizinischer Anwendungen, z.B. für Diagnose- und Therapiezwecke, z.B. bei Untersuchungen oder im chirurgischen Bereich.

Gemäß einer vorteilhaften Ausgestaltung der Erfindung ist vorgesehen, dass auf dem Display der Videobild-Übertragungseinrichtung eine Netzwerk-Adresse eines in einem Netzwerk datenübertragenden Geräts visualisiert wird, z.B. die eigene Netzwerkadresse der Videobild-Übertragungseinrichtung, und diese Netzwerkadresse durch Scannen mittels der Augmented Reality-, Virtual Reality- oder Mixed Reality-Anzeigeeinrichtung erfasst und automatisch ausgewertet wird. Auf diese Weise kann der Anwender sehr einfach und ohne Vorkenntnisse die Kopplung seiner AR-Anzeigeeinrichtung mit der Videobild-Übertragungseinrichtung und damit mit der Videobildquelle bewerkstelligen.

Die eingangs genannte Aufgabe wird außerdem gelöst durch ein Computerprogramm mit Programmcodemitteln, eingerichtet zur Durchführung eines Verfahrens der zuvor erläuterten Art, wenn das Computerprogramm auf einem Rechner ausgeführt wird. Auch hierdurch können die zuvor erläuterten Vorteile realisiert werden. Der Rechner kann z.B. ein Rechner der Verarbeitungseinrichtung der Videobild-Übertragungseinrichtung sein.

Im Sinne der vorliegenden Erfindung ist unter dem unbestimmten Begriff "ein" kein Zahlwort zu verstehen. Wenn also z.B. von einem Bauteil die Rede ist, so ist dies im Sinne von "mindestens einem Bauteil" zu interpretieren. Soweit ein Rechner erwähnt ist, kann dieser dazu eingerichtet sein, ein Computerprogramm, z.B. im Sinne von Software, auszuführen. Der Rechner kann als handelsüblicher Computer ausgebildet sein, z.B. als PC, Laptop, Notebook, Tablet oder Smartphone, oder als Mikroprozessor, Mikrocontroller oder FPGA, oder als Kombination aus solchen Elementen.

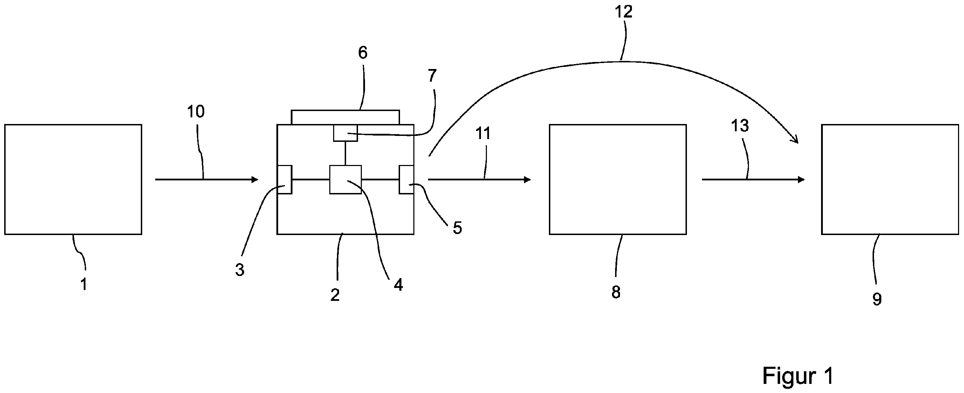

Die Erfindung wird nachfolgend anhand eines Ausführungsbeispiels unter Verwendung der Zeichnung Figur 1 näher erläutert.

Die Figur 1 zeigt eine Anordnung zur Live-Übertragung von Videobildsequenzen von einer Videobildquelle 1 auf eine AR-Anzeigeeinrichtung 9. Die Videobildquelle 1 ist ausgebildet als bildgebendes medizinisches Untersuchungsgerät. Dies ist für Anwendungen im medizinischen Bereich vorteilhaft. Das bildgebende medizinische Untersuchungsgerät kann z.B. ein Endoskop oder ein Mikroskop sein. Die AR-Anzeigeeinrichtung 9 kann eine grundsätzlich beliebige AR-Anzeigeeinrichtung sein, z.B. die Microsoft HoloLens.

Die Figur 1 zeigt eine Videobild-Übertragungseinrichtung 2, die einen Eingang 3, eine Verarbeitungseinrichtung 4, einen Ausgang 5, ein Display 6 und eine Displayansteuerung 7 aufweist. Der Eingang 3 ist zur Zuführung zu übertragender Videobildsequenzen von der Videobildquelle 1 zu der Verarbeitungseinrichtung 4 eingerichtet. Zu diesem Zweck kann der Eingang 3 über einen Datenkanal 10 mit der Videobildquelle 1 gekoppelt sein. Der Datenkanal 10 ist ein drahtgebundener Übertragungskanal, z.B. ein HDMI-Kabel.

Die Verarbeitungseinrichtung ist zur Live-Umwandlung der über den Eingang 3 empfangenen Videobildsequenzen in auszugebende Videobildsequenzen eingerichtet, die über den Ausgang 5 ausgegeben werden. Der Ausgang 5 ist zur Ausgabe der auszugebenden Videobildsequenzen speziell an eine AR-Anzeigeeinrichtung 9 eingerichtet. Für die Datenübertragung zur AR-Anzeigeeinrichtung 9 kann der Ausgang 5 entweder über einen Datenkanal 12 direkt oder indirekt über einen Datenkanal 11, eine Übertragungseinheit 8 und einen Datenkanal 13 mit der AR-Anzeigeeinrichtung 9 gekoppelt sein. Die Datenübertragung über die Datenkanäle 12, 13 erfolgt bevorzugt drahtlos, z.B. über ein WLAN-Datenprotokoll. Die Datenübertragung über den Datenkanal 11 kann ebenfalls drahtlos erfolgen, sie kann auch drahtgebunden erfolgen.

Das Übertragungsgerät 8 sorgt für eine weite Übertragung der vom Datenkanal 11 empfangenen Daten auf den Datenkanal 13 und damit zur AR-Anzeigeeinrichtung 9. Beispielsweise kann das Übertragungsgerät 8 als Router oder als sonstige Relaisstation in einem drahtlosen Übertragungsnetz, z.B. einem WLAN-Netz, ausgebildet sein.

Die Videobild-Übertragungseinrichtung 2 ist zudem dazu eingerichtet, über die Displayansteuerung 7 auf dem Display 6 Betriebsdaten der Videobild-Übertragungseinrichtung 2 zu visualisieren, z.B. eine Netzwerk-Adresse der Videobild-Übertragungseinrichtung 2.

## Patentansprüche

1. Videobild-Übertragungseinrichtung (2) zur Live-Übertragung von Videobildsequenzen mittels Streaming von einer Videobildquelle auf eine Augmented Reality-, Virtual Reality- oder Mixed Reality-Anzeigeeinrichtung (9), wobei die Videobild-Übertragungseinrichtung (2)
a) einen Eingang (3) aufweist, der zur Zuführung zu übertragender Videobildsequenzen von der Videobildquelle (1) zu einer Verarbeitungseinrichtung (4) der Videobild-Übertragungseinrichtung (2) eingerichtet ist,
b) die Verarbeitungseinrichtung (4) aufweist, die zur Live-Umwandlung der über den Eingang (3) empfangenen Videobildsequenzen in auszugebende Videobildsequenzen eingerichtet ist, und
c) einen Ausgang (5) aufweist, der zur Ausgabe der auszugebenden Videobildsequenzen an die Augmented Reality-, Virtual Reality- oder Mixed Reality-Anzeigeeinrichtung (9) eingerichtet ist,
wobei der Eingang (3) dazu eingerichtet ist, die zu übertragenden Videobildsequenzen von einem bildgebenden medizinischen Untersuchungsgerät als Videobildquelle zu empfangen.

2. Videobild-Übertragungseinrichtung nach Anspruch 1, **dadurch gekennzeichnet, dass** der Ausgang (5) als drahtlose Schnittstelle, insbesondere als WLAN-Schnittstelle, ausgebildet ist.

3. Videobild-Übertragungseinrichtung nach einem der vorhergehenden Ansprüche, **dadurch gekennzeichnet, dass** die Verarbeitungseinrichtung (4) wenigstens einen Codierungskonverter aufweist, der dazu eingerichtet ist, über den Eingang empfangene Videobildsequenzen einer ersten Codierung in auszugebende Videobildsequenzen einer zweiten Codierung, die sich von der ersten Codierung unterscheidet, zu konvertieren.

4. Videobild-Übertragungseinrichtung nach einem der vorhergehenden Ansprüche, **dadurch gekennzeichnet, dass** die Videobild-Übertragungseinrichtung (2) wenigstens ein Display (6) und eine Displayansteuerung (7) aufweist, die dazu eingerichtet ist, auf dem Display (6) Betriebsdaten der Videobild-Übertragungseinrichtung (2) zu visualisieren.

5. Videobild-Übertragungseinrichtung nach Anspruch 4, **dadurch gekennzeichnet, dass** die Displayansteuerung (7) dazu eingerichtet ist, auf dem Display (6) die eigene Netzwerkadresse der Videobild-Übertragungseinrichtung (2) und/oder eine Netzwerk-Adresse der Augmented Reality-, Virtual Reality- oder Mixed Reality-Anzeigeeinrichtung (9), auf die auszugebenden Videobildsequenzen übertragen werden sollen, im Klartext und/oder in einer maschinenlesbaren Codierung zu visualisieren.

6. Videobild-Übertragungseinrichtung nach einem der vorhergehenden Ansprüche, **dadurch gekennzeichnet, dass** der Eingang (3) dazu eingerichtet ist, Videobildsequenzen von einem Endoskop oder einem Mikroskop als Videobildquelle zu empfangen.

7. Verfahren zur Live-Übertragung von Videobildsequenzen mittels Streaming von einer Videobildquelle (1) auf eine Augmented Reality-, Virtual Reality- oder Mixed Reality-Anzeigeeinrichtung (9) unter Verwendung einer Videobild-Übertragungseinrichtung (2), wobei die Videobild-Übertragungseinrichtung (2)
a) einen Eingang (3) aufweist, der zur Zuführung zu übertragender Videobildsequenzen von der Videobildquelle (1) zu einer Verarbeitungseinrichtung (4) der Videobild-Übertragungseinrichtung (2) eingerichtet ist,
b) die Verarbeitungseinrichtung (4) aufweist, die zur Live-Umwandlung der über den Eingang (3) empfangenen Videobildsequenzen in auszugebende Videobildsequenzen eingerichtet ist, und
c) einen Ausgang (5) aufweist, der zur Ausgabe der auszugebenden Videobildsequenzen an die Augmented Reality-, Virtual Reality- oder Mixed Reality-Anzeigeeinrichtung (9) eingerichtet ist,
wobei der Eingang (3) dazu eingerichtet ist, die zu übertragenden Videobildsequenzen von einem bildgebenden medizinischen Untersuchungsgerät als Videobildquelle zu empfangen.

8. Verfahren nach Anspruch 7, **dadurch gekennzeichnet, dass** der Ausgang (5) als drahtlose Schnittstelle, insbesondere als WLAN-Schnittstelle, ausgebildet ist.

9. Verfahren nach Anspruch 7 oder 8, **dadurch gekennzeichnet, dass** die Verarbeitungseinrichtung (4) wenigstens einen Codierungskonverter aufweist, der dazu eingerichtet ist, über den Eingang empfangene Videobildsequenzen einer ersten Codierung in auszugebende Videobildsequenzen einer zweiten Codierung, die sich von der ersten Codierung unterscheidet, zu konvertieren.

10. Verfahren nach einem der Ansprüche 7 bis 9, **dadurch gekennzeichnet, dass** die Übertragung der Videobildsequenzen von der Videobildquelle (1) zum Eingang (3) und/oder vom Ausgang (5) zu der Augmented Reality-, Virtual Reality- oder Mixed Reality-Anzeigeeinrichtung (9) im VSI-Format erfolgt.

11. Verfahren nach einem der Ansprüche 7 bis 10, **dadurch gekennzeichnet, dass** die Videobild-Übertragungseinrichtung (2) wenigstens ein Display (6) und eine Displayansteuerung (7) aufweist, die dazu eingerichtet ist, auf dem Display (6) Betriebsdaten der Videobild-Übertragungseinrichtung (2) zu visualisieren.

12. Verfahren nach einem der Ansprüche 7 bis 11, **dadurch gekennzeichnet, dass** die Displayansteuerung (7) dazu eingerichtet ist, auf dem Display (6) die eigene Netzwerkadresse der Videobild-Übertragungseinrichtung (2) und/oder eine Netzwerk-Adresse der Augmented Reality-, Virtual Reality- oder Mixed Reality-Anzeigeeinrichtung (9), auf die auszugebenden Videobildsequenzen übertragen werden sollen, im Klartext und/oder in einer maschinenlesbaren Codierung zu visualisieren.

13. Verfahren nach einem der Ansprüche 11 bis 12, **dadurch gekennzeichnet, dass** auf dem Display (6) der Videobild-Übertragungseinrichtung (2) eine Netzwerk-Adresse eines in einem Netzwerk Daten übertragenden Geräts visualisiert wird und diese Netzwerkadresse durch Scannen mittels der Augmented Reality-, Virtual Reality- oder Mixed Reality-Anzeigeeinrichtung (9) erfasst und automatisch ausgewertet wird.

14. Verfahren nach einem der Ansprüche 7 bis 13, **dadurch gekennzeichnet, dass** der Eingang (3) dazu eingerichtet ist, Videobildsequenzen von einem Endoskop oder einem Mikroskop als Videobildquelle zu empfangen.

15. Computerprogramm mit Programmcodemitteln, eingerichtet zur Durchführung eines Verfahrens nach einem der Ansprüche 7 bis 14, wenn das Computerprogramm auf einem Rechner ausgeführt wird.

## Geänderte Patentansprüche

### Geänderte Patentansprüche gemäss Regel 137(2) EPÜ.

**1.** Videobild-Übertragungseinrichtung (2) zur Live-Übertragung von Videobildsequenzen mittels Streaming von einer Videobildquelle (1) auf eine Augmented Reality-, Virtual Reality- oder Mixed Reality-Anzeigeeinrichtung (9), wobei
a) die Videobild-Übertragungseinrichtung (2) einen Eingang (3) aufweist, der dazu eingerichtet ist, mit der Videobildquelle (1) gekoppelt zu werden und der zur Zuführung zu übertragender Videobildsequenzen von der Videobildquelle (1) zu einer Verarbeitungseinrichtung (4) der Videobild-Übertragungseinrichtung (2) eingerichtet ist, und
b) die Videobild-Übertragungseinrichtung (2) die Verarbeitungseinrichtung (4) aufweist, die zur Live-Umwandlung der über den Eingang (3) empfangenen Videobildsequenzen in auszugebende Videobildsequenzen eingerichtet ist und wenigstens einen Codierungskonverter aufweist, der dazu eingerichtet ist, über den Eingang empfangene Videobildsequenzen einer ersten Codierung in auszugebende Videobildsequenzen einer zweiten Codierung, die sich von der ersten Codierung unterscheidet, zu konvertieren, und
c) die Videobild-Übertragungseinrichtung (2) einen Ausgang (5) aufweist, der dazu eingerichtet ist, mit der Augmented Reality-, Virtual Reality- oder Mixed Reality-Anzeigeeinrichtung (9) gekoppelt zu werden und der zur Ausgabe der auszugebenden Videobildsequenzen an die Augmented Reality-, Virtual Reality- oder Mixed Reality-Anzeigeeinrichtung (9) eingerichtet ist, und
d) die Videobild-Übertragungseinrichtung (2) separat von dem bildgebenden medizinischen Untersuchungsgerät und separat von der Augmented Reality-, Virtual Reality- oder Mixed Reality-Anzeigeeinrichtung (9) ausgebildet ist und ein Gehäuse aufweist, in das der Eingang (3), die Verarbeitungseinrichtung (4) und der Ausgang (5) integriert sind, und
e) der Eingang (3) dazu eingerichtet ist, die zu übertragenden Videobildsequenzen von einem bildgebenden medizinischen Untersuchungsgerät als Videobildquelle (1) zu empfangen.

**2.** Videobild-Übertragungseinrichtung nach Anspruch 1, **dadurch gekennzeichnet, dass** der Ausgang (5) als drahtlose Schnittstelle, insbesondere als WLAN-Schnittstelle, ausgebildet ist.

**3.** Videobild-Übertragungseinrichtung nach einem der vorhergehenden Ansprüche, **dadurch gekennzeichnet, dass** die Videobild-Übertragungseinrichtung (2) wenigstens ein Display (6) und eine Displayansteuerung (7) aufweist, die dazu eingerichtet ist, auf dem Display (6) Betriebsdaten der Videobild-Übertragungseinrichtung (2) zu visualisieren.

**4.** Videobild-Übertragungseinrichtung nach Anspruch 3, **dadurch gekennzeichnet, dass** die Displayansteuerung (7) dazu eingerichtet ist, auf dem Display (6) die eigene Netzwerkadresse der Videobild-Übertragungseinrichtung (2) und/oder eine Netzwerk-Adresse der Augmented Reality-, Virtual Reality- oder Mixed Reality-Anzeigeeinrichtung (9), auf die auszugebenden Videobildsequenzen übertragen werden sollen, im Klartext und/oder in einer maschinenlesbaren Codierung zu visualisieren.

**5.** Videobild-Übertragungseinrichtung nach einem der vorhergehenden Ansprüche, **dadurch gekennzeichnet, dass** der Eingang (3) dazu eingerichtet ist, Videobildsequenzen von einem Endoskop oder einem Mikroskop als Videobildquelle zu empfangen.

**6.** Verfahren zur Live-Übertragung von Videobildsequenzen mittels Streaming von einer Videobildquelle (1) auf eine Augmented Reality-, Virtual Reality- oder Mixed Reality-Anzeigeeinrichtung (9) unter Verwendung einer Videobild-Übertragungseinrichtung (2), wobei
a) die Videobild-Übertragungseinrichtung (2) einen Eingang (3) aufweist, der dazu eingerichtet ist, mit der Videobildquelle (1) gekoppelt zu werden und der zur Zuführung zu übertragender Videobildsequenzen von der Videobildquelle (1) zu einer Verarbeitungseinrichtung (4) der Videobild-Übertragungseinrichtung (2) eingerichtet ist, und
b) die Videobild-Übertragungseinrichtung (2) die Verarbeitungseinrichtung (4) aufweist, die zur Live-Umwandlung der über den Eingang (3) empfangenen Videobildsequenzen in auszugebende Videobildsequenzen eingerichtet ist und wenigstens einen Codierungskonverter aufweist, der dazu eingerichtet ist, über den Eingang empfangene Videobildsequenzen einer ersten Codierung in auszugebende Videobildsequenzen einer zweiten Codierung, die sich von der ersten Codierung unterscheidet, zu konvertieren, und
c) die Videobild-Übertragungseinrichtung (2) einen Ausgang (5) aufweist, der dazu eingerichtet ist, mit der Augmented Reality-, Virtual Reality- oder Mixed Reality-Anzeigeeinrichtung (9) gekoppelt zu werden und der zur Ausgabe der auszugebenden Videobildsequenzen an die Augmented Reality-, Virtual Reality- oder Mixed Reality-Anzeigeeinrichtung (9) eingerichtet ist, und
d) die Videobild-Übertragungseinrichtung (2) separat von dem bildgebenden medizinischen Untersuchungsgerät und separat von der Augmented Reality-, Virtual Reality- oder Mixed Reality-Anzeigeeinrichtung (9) ausgebildet ist und ein Gehäuse aufweist, in das der Eingang (3), die Verarbeitungseinrichtung (4) und der Ausgang (5) integriert sind, und
e) der Eingang (3) dazu eingerichtet ist, die zu übertragenden Videobildsequenzen von einem bildgebenden medizinischen Untersuchungsgerät als Videobildquelle (1) zu empfangen.

**7.** Verfahren nach Anspruch 6, **dadurch gekennzeichnet, dass** der Ausgang (5) als drahtlose Schnittstelle, insbesondere als WLAN-Schnittstelle, ausgebildet ist.

**8.** Verfahren nach Anspruch 6 oder 7, **dadurch gekennzeichnet, dass** die Übertragung der Videobildsequenzen von der Videobildquelle (1) zum Eingang (3) und/oder vom Ausgang (5) zu der Augmented Reality-, Virtual Reality- oder Mixed Reality-Anzeigeeinrichtung (9) im VSI-Format erfolgt.

**9.** Verfahren nach einem der Ansprüche 6 bis 8, **dadurch gekennzeichnet, dass** die Videobild-Übertragungseinrichtung (2) wenigstens ein Display (6) und eine Displayansteuerung (7) aufweist, die dazu eingerichtet ist, auf dem Display (6) Betriebsdaten der Videobild-Übertragungseinrichtung (2) zu visualisieren.

**10.** Verfahren nach einem der Ansprüche 6 bis 9, **dadurch gekennzeichnet, dass** die Displayansteuerung (7) dazu eingerichtet ist, auf dem Display (6) die eigene Netzwerkadresse der Videobild-Übertragungseinrichtung (2) und/oder eine Netzwerk-Adresse der Augmented Reality-, Virtual Reality- oder Mixed Reality-Anzeigeeinrichtung (9), auf die auszugebenden Videobildsequenzen übertragen werden sollen, im Klartext und/oder in einer maschinenlesbaren Codierung zu visualisieren.

**11.** Verfahren nach einem der Ansprüche 9 bis 10, **dadurch gekennzeichnet, dass** auf dem Display (6) der Videobild-Übertragungseinrichtung (2) eine Netzwerk-Adresse eines in einem Netzwerk Daten übertragenden Geräts visualisiert wird und diese Netzwerkadresse durch Scannen mittels der Augmented Reality-, Virtual Reality- oder Mixed Reality-Anzeigeeinrichtung (9) erfasst und automatisch ausgewertet wird.

**12.** Verfahren nach einem der Ansprüche 6 bis 11, **dadurch gekennzeichnet, dass** der Eingang (3) dazu eingerichtet ist, Videobildsequenzen von einem Endoskop oder einem Mikroskop als Videobildquelle zu empfangen.

**13.** Computerprogramm mit Programmcodemitteln, eingerichtet zur Durchführung eines Verfahrens nach einem der Ansprüche 6 bis 12, wenn das Computerprogramm auf einem Rechner ausgeführt wird.

**1.** Videobild-Übertragungseinrichtung (2) zur Live-Übertragung von Videobildsequenzen mittels Streaming von einer Videobildquelle (1) auf eine Augmented Reality-, Virtual Reality- oder Mixed Reality-Anzeigeeinrichtung (9), wobei
a) die Videobild-Übertragungseinrichtung (2) einen Eingang (3) aufweist, der dazu eingerichtet ist, mit der Videobildquelle (1) gekoppelt zu werden und der zur Zuführung zu übertragender Videobildsequenzen von der Videobildquelle (1) zu einer Verarbeitungseinrichtung (4) der Videobild-Übertragungseinrichtung (2) eingerichtet ist, und
b) die Videobild-Übertragungseinrichtung (2) die Verarbeitungseinrichtung (4) aufweist, die zur Live-Umwandlung der über den Eingang (3) empfangenen Videobildsequenzen in auszugebende Videobildsequenzen eingerichtet ist und wenigstens einen Codierungskonverter aufweist, der dazu eingerichtet ist, über den Eingang empfangene Videobildsequenzen einer ersten Codierung in auszugebende Videobildsequenzen einer zweiten Codierung, die sich von der ersten Codierung unterscheidet, zu konvertieren, und
c) die Videobild-Übertragungseinrichtung (2) einen Ausgang (5) aufweist, der dazu eingerichtet ist, mit der Augmented Reality-, Virtual Reality- oder Mixed Reality-Anzeigeeinrichtung (9) gekoppelt zu werden und der zur Ausgabe der auszugebenden Videobildsequenzen an die Augmented Reality-, Virtual Reality- oder Mixed Reality-Anzeigeeinrichtung (9) eingerichtet ist, und
d) die Videobild-Übertragungseinrichtung (2) separat von dem bildgebenden medizinischen Untersuchungsgerät und separat von der Augmented Reality-, Virtual Reality- oder Mixed Reality-Anzeigeeinrichtung (9) ausgebildet ist als handliche kleine Box, die vom Anwender leicht transportiert werden kann, und
e) der Eingang (3) dazu eingerichtet ist, die zu übertragenden Videobildsequenzen von einem bildgebenden medizinischen Untersuchungsgerät als Videobildquelle (1) zu empfangen.

**2.** Videobild-Übertragungseinrichtung nach Anspruch 1, **dadurch gekennzeichnet, dass** der Ausgang (5) als drahtlose Schnittstelle, insbesondere als WLAN-Schnittstelle, ausgebildet ist.

**3.** Videobild-Übertragungseinrichtung nach einem der vorhergehenden Ansprüche, **dadurch gekennzeichnet, dass** die Videobild-Übertragungseinrichtung (2) wenigstens ein Display (6) und eine Displayansteuerung (7) aufweist, die dazu eingerichtet ist, auf dem Display (6) Betriebsdaten der Videobild-Übertragungseinrichtung (2) zu visualisieren.

**4.** Videobild-Übertragungseinrichtung nach Anspruch 3, **dadurch gekennzeichnet, dass** die Displayansteuerung (7) dazu eingerichtet ist, auf dem Display (6) die eigene Netzwerkadresse der Videobild-Übertragungseinrichtung (2) und/oder eine Netzwerk-Adresse der Augmented Reality-, Virtual Reality- oder Mixed Reality-Anzeigeeinrichtung (9), auf die auszugebenden Videobildsequenzen übertragen werden sollen, im Klartext und/oder in einer maschinenlesbaren Codierung zu visualisieren.

**5.** Videobild-Übertragungseinrichtung nach einem der vorhergehenden Ansprüche, **dadurch gekennzeichnet, dass** der Eingang (3) dazu eingerichtet ist, Videobildsequenzen von einem Endoskop oder einem Mikroskop als Videobildquelle zu empfangen.

**6.** Verfahren zur Live-Übertragung von Videobildsequenzen mittels Streaming von einer Videobildquelle (1) auf eine Augmented Reality-, Virtual Reality- oder Mixed Reality-Anzeigeeinrichtung (9) unter Verwendung einer Videobild-Übertragungseinrichtung (2), wobei
a) die Videobild-Übertragungseinrichtung (2) einen Eingang (3) aufweist, der dazu eingerichtet ist, mit der Videobildquelle (1) gekoppelt zu werden und der zur Zuführung zu übertragender Videobildsequenzen von der Videobildquelle (1) zu einer Verarbeitungseinrichtung (4) der Videobild-Übertragungseinrichtung (2) eingerichtet ist, und
b) die Videobild-Übertragungseinrichtung (2) die Verarbeitungseinrichtung (4) aufweist, die zur Live-Umwandlung der über den Eingang (3) empfangenen Videobildsequenzen in auszugebende Videobildsequenzen eingerichtet ist und wenigstens einen Codierungskonverter aufweist, der dazu eingerichtet ist, über den Eingang empfangene Videobildsequenzen einer ersten Codierung in auszugebende Videobildsequenzen einer zweiten Codierung, die sich von der ersten Codierung unterscheidet, zu konvertieren, und
c) die Videobild-Übertragungseinrichtung (2) einen Ausgang (5) aufweist, der dazu eingerichtet ist, mit der Augmented Reality-, Virtual Reality- oder Mixed Reality-Anzeigeeinrichtung (9) gekoppelt zu werden und der zur Ausgabe der auszugebenden Videobildsequenzen an die Augmented Reality-, Virtual Reality- oder Mixed Reality-Anzeigeeinrichtung (9) eingerichtet ist, und
d) die Videobild-Übertragungseinrichtung (2) separat von dem bildgebenden medizinischen Untersuchungsgerät und separat von der Augmented Reality-, Virtual Reality- oder Mixed Reality-Anzeigeeinrichtung (9) ausgebildet ist als handliche kleine Box, die vom Anwender leicht transportiert werden kann, und
e) der Eingang (3) dazu eingerichtet ist, die zu übertragenden Videobildsequenzen von einem bildgebenden medizinischen Untersuchungsgerät als Videobildquelle (1) zu empfangen.

**7.** Verfahren nach Anspruch 6, **dadurch gekennzeichnet, dass** der Ausgang (5) als drahtlose Schnittstelle, insbesondere als WLAN-Schnittstelle, ausgebildet ist.

**8.** Verfahren nach Anspruch 6 oder 7, **dadurch gekennzeichnet, dass** die Übertragung der Videobildsequenzen von der Videobildquelle (1) zum Eingang (3) und/oder vom Ausgang (5) zu der Augmented Reality-, Virtual Reality- oder Mixed Reality-Anzeigeeinrichtung (9) im VSI-Format erfolgt.

**9.** Verfahren nach einem der Ansprüche 6 bis 8, **dadurch gekennzeichnet, dass** die Videobild-Übertragungseinrichtung (2) wenigstens ein Display (6) und eine Displayansteuerung (7) aufweist, die dazu eingerichtet ist, auf dem Display (6) Betriebsdaten der Videobild-Übertragungseinrichtung (2) zu visualisieren.

**10.** Verfahren nach einem der Ansprüche 6 bis 9, **dadurch gekennzeichnet, dass** die Displayansteuerung (7) dazu eingerichtet ist, auf dem Display (6) die eigene Netzwerkadresse der Videobild-Übertragungseinrichtung (2) und/oder eine Netzwerk-Adresse der Augmented Reality-, Virtual Reality- oder Mixed Reality-Anzeigeeinrichtung (9), auf die auszugebenden Videobildsequenzen übertragen werden sollen, im Klartext und/oder in einer maschinenlesbaren Codierung zu visualisieren.

**11.** Verfahren nach einem der Ansprüche 9 bis 10, **dadurch gekennzeichnet, dass** auf dem Display (6) der Videobild-Übertragungseinrichtung (2) eine Netzwerk-Adresse eines in einem Netzwerk Daten übertragenden Geräts visualisiert wird und diese Netzwerkadresse durch Scannen mittels der Augmented Reality-, Virtual Reality- oder Mixed Reality-Anzeigeeinrichtung (9) erfasst und automatisch ausgewertet wird.

**12.** Verfahren nach einem der Ansprüche 6 bis 11, **dadurch gekennzeichnet, dass** der Eingang (3) dazu eingerichtet ist, Videobildsequenzen von einem Endoskop oder einem Mikroskop als Videobildquelle zu empfangen.

**13.** Computerprogramm mit Programmcodemitteln, eingerichtet zur Durchführung eines Verfahrens nach einem der Ansprüche 6 bis 12, wenn das Computerprogramm auf einem Rechner ausgeführt wird.
